(19) Europäisches Patentamt
European Patent Office
Office européen des brevets

(11) Veröffentlichungsnummer: **0 193 709**
**A2**

(12) # EUROPÄISCHE PATENTANMELDUNG

(21) Anmeldenummer: **86100461.2**

(22) Anmeldetag: **15.01.86**

(51) Int. Cl.⁴: **C 07 C 15/20**, C 07 C 69/74, C 07 D 493/04, C 07 D 487/04

(30) Priorität: **06.03.85 DE 3507878**

(43) Veröffentlichungstag der Anmeldung: **10.09.86 Patentblatt 86/37**

(84) Benannte Vertragsstaaten: **AT BE CH DE FR GB IT LI NL SE**

(71) Anmelder: **HÜLS AKTIENGESELLSCHAFT, Patentabteilung / PB 15 - Postfach 13 20, D-4370 Marl 1 (DE)**

(72) Erfinder: **Bartmann, Martin, Dr., Burgstrasse 35, D-4350 Recklinghausen (DE)**

(54) **Substituierte Octahydroanthracene und Verfahren zu ihrer Herstellung.**

(57) Die vorliegende Erfindung betrifft ein Verfahren zur Herstellung von 1,2,3,4,5,6,7,8-Octahydroanthracenen, die wenigstens in zwei der Positionen 2, 3, 6, 7, 9 und 10 substituiert sind, durch Umsetzung von Benzodicyclobutenen I mit trans-Olefinen II, cis-Olefinen III und/oder cyclischen Olefinen IV bei Temperaturen oberhalb von 100 °C sowie Reaktionsprodukte, die nach diesem Verfahren erhältlich sind.

EP 0 193 709 A2

ACTORUM AG

Substituierte Octahydroanthracene und Verfahren zu ihrer Herstellung

Polyfunktionelle Benzo-[1.2:4.5]-dicyclohexene, die auch als 1,2,3,4,5,6,7,8-Octahydroanthracene bezeichnet werden können, sind unter anderem als Ausgangspunkt für die Herstellung von hochtemperaturbeständigen Polyestern von Interesse. Mit Ausnahme des 1,2,3,4,5,6,7,8-Octahydroanthracen-2,3,6,7-tetracarbonsäureanhydrids, das aus dem entsprechenden Cyclohexadienderivat gewonnen wurde (vgl. D. T. Longone, F.-P. Boettcher, Journal of the Am. Chemical Society 85, 3436 ff. (1963), sind derartige Verbindungen nicht bekannt, und es gibt bisher kein direktes Verfahren zu ihrer Herstellung.

Es wurde jetzt gefunden, daß 1,2,3,4,5,6,7,8-Octahydroanthracene, die wenigstens in zwei der Positionen 2, 3, 6, 7, 9 und 10 substituiert sind, durch Diels-Alder-Reaktion von Benzodicyclobutenen mit den entsprechenden substituierten Olefinen leicht zugänglich sind.

Das glatte Reaktionsverhalten der Benzodicyclobutene ist überraschend. Zwar war bekannt, daß Benzocyclobutene Dienreaktivität besitzen (siehe I. L. Klundt, Chem. Rev. 70, 471 (1970) und W. Oppholzer, Angew. Chem. 89, 10 (1977) und auch 1,2,4,5-Tetraalkylidencyclohexane lassen sich mit Maleinsäureanhydriden abfangen; im Falle der Benzodicyclobutene steht die reaktive Dienform a) aber in Mesomerie mit Grenzstrukturen wie b) und c), die radikalischen Charakter besitzen und daher ganz anders reagieren sollten.

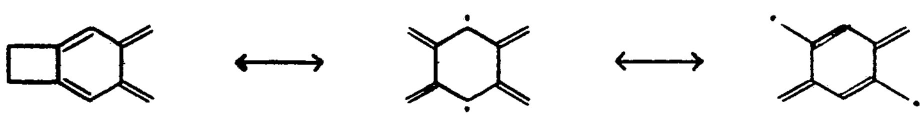

a) b) c)

Überraschenderweise kommt es jedoch nicht zu solchen störenden Nebenreaktionen.

Gegenstand der vorliegenden Erfindung sind die Verfahren zur Herstellung der Octahydroanthracene nach den Ansprüchen 1 bis 4 sowie die Verbindungen nach den Ansprüchen 5 bis 7.

Die erfindungsgemäß einsetzbaren Benzodicyclobutene I weisen die Formel

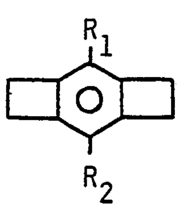

auf, wobei $R_1$ und $R_2$ jeweils für ein Wasserstoffatom, ein Halogen, insbesondere Chlor und Brom, oder für eine Alkylgruppe mit 1 bis 4 C-Atomen, insbesondere die Methylgruppe, stehen können. Sie können nach den in der Literatur beschriebenen Methoden hergestellt werden (vgl. J. Am. Chem. Soc. <u>100</u>, 2892 (1978), J. Org. Chem. <u>46</u>, 4608 (1981), J. Am. Chem. Soc. <u>82</u>, 2524 (1960), Tetrah. Lett. <u>46</u>, 4569 (1978). Vorzugsweise wird Benzodicyclobuten selbst eingesetzt.

Als erfindungsgemäß einsetzbare substituierte Olefine kommen infrage:

II trans-Olefine der allgemeinen Formel

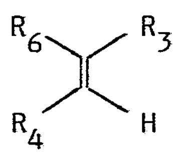

wobei $R_3$ für einen Rest der Formel $-CO_2R_5$ oder 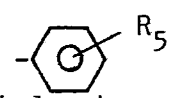 steht und $R_5$ die Bedeutung von Wasserstoff oder eines Alkylrestes mit 1 bis 4 C-Atomen hat und vorzugsweise Methyl oder Ethyl ist, $R_4$ für einen der bereits durch $R_3$ oder $R_5$ definierten Reste steht, und wobei $R_6$ für Wasserstoff oder die Methylgruppe steht. Bevorzugt ist Fumarsäurediethylester.

III cis-Olefine der allgemeinen Formel

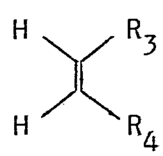

mit den gleichen Bedeutungen für $R_3$ und $R_4$ wie vorstehend angegeben. Bevorzugt sind Zimtsäure- und Acrylsäureethylester.

IV cyclische Olefine der allgemeinen Formel

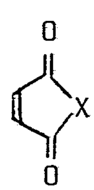 mit X = O, NPh, $NR_5$,

wobei $R_5$ wiederum die bereits vorstehend aufgeführte Bedeutung hat. Bevorzugt ist Maleinsäureanhydrid.

Um Nebenreaktionen auszuschließen, sollte das eingesetzte Olefin nur eine Kohlenstoff-Kohlenstoff-Doppelbindung pro Molekül enthalten. Selbstverständlich können auch Gemische unterschiedlicher Olefine und/ oder Benzodicyclobutene eingesetzt werden.

Die Reaktion zwischen dem substituierten Olefin und dem Benzodicyclobuten wird bei Temperaturen oberhalb von 100 °C, bevorzugt bei Temperaturen zwischen 170 und 250 °C, durchgeführt.

Bezogen auf 1 Mol des Benzodicyclobutens I sollten mindestens 2 Mol der Olefinkomponente eingesetzt werden. Bei unter Normalbedingungen flüssigen Olefinen kann ein hoher Olefinüberschuß,zweckmäßigerweise bis zu 100 Mol, eingesetzt werden.

Bei festen, hochschmelzenden Ausgangsstoffen führt man die Reaktion bevorzugt in Lösemitteln durch, die gegenüber dem Olefin, dem Benzodicyclobuten und dem Reaktionsprodukt inert sind. Vorteilhaft sind Lösemittel mit Siedepunkten über 100 °C, wie z. B. Sulfolan (Tetrahydrothiophendioxid), Dichlorbenzol, Diphenylether oder Phthalsäurediethylester. Lösemittel mit einem Siedepunkt unter 100 °C, z. B. Chloroform oder Dichlormethan, können verwendet werden, wenn der Druck im System über Normaldruck hinaus erhöht wird.

Es hat sich als günstig erwiesen, die erfindungsgemäßen Reaktionen unter Bedingungen durchzuführen, die die radikalische Polymerisation der eingesetzten Olefine verhindern. Beispielsweise können geringe Mengen von Stabilisatoren auf Basis von sterisch gehinderten Phenolen oder Hydrochinonen zugesetzt werden.

Die erhaltenen Produkte fallen stets als Isomerengemisch an. Bedingt durch die sterischen Verhältnisse ist im Falle des Einsatzes cyclischer Olefine IV mit einem Gemisch folgender cis- und trans-Produkte zu rechnen:

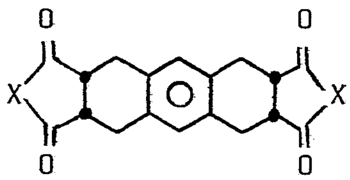
sowie
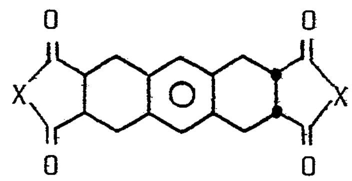

Im Falle des Einsatzes der cis-Olefine III etwa ist zusätzlich folgende Isomerie möglich:

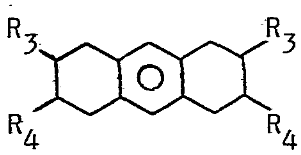
sowie
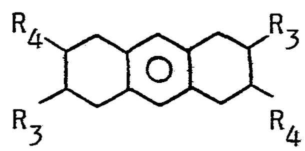

Damit wird auch das Auftreten von 4 Isomeren bei der Umsetzung mit Zimtsäureethylester erklärt.

Beispiel 1

Synthese des 1,2,3,4,5,6,7,8-Octahydroanthracen-2,3,6,7-tetracarbonsäuretetraethylesters (Isomerengemisch)

Unter Stickstoffabdeckung rührt man das Gemisch aus
20 g Fumarsäurediethylester,
1 g Benzo-[1.2:4.5]-dicyclobuten und
10 mg Hydrochinon
6 Stunden bei 210 °C und trennt anschließend den Überschuß des Fumarsäurediethylesters destillativ ab. Den Rückstand kristallisiert man aus wäßrigem Aceton um.

Ausbeute : 3,1 g (85 % d. Th.)
Schmelzpunkt: 126 - 140 °C
NMR-Spektrum: $\delta$ = 6,8 2H (s)
= 4,1 8H (Quartett)
= 2,9 12H (m)
= 1,25 12H (Triplett)

Im Massenspektrum wurde die theoretische Molmasse 474 bestätigt.

Beispiel 2

9,10-Dimethyl-1,2,3,4,5,6,7,8-octahydroanthracen-2,3,6,7-tetracarbonsäuredianhydrid

Die Lösung von
1 g 3,6-Dimethylbenzol-[1.2:4.5]-dicyclobuten,
2,5 g Maleinsäureanhydrid und
10 mg 2,6-Di-tert.-butyl-p-kresol
in 20 ml Sulfolan wird 6 Stunden bei 230 °C unter Stickstoffabdeckung gerührt. Anschließend trennt man das Lösemittel und das überschüssige Maleinsäureanhydrid destillativ ab. Den Rückstand kristallisiert man aus Toluol um.

Ausbeute : 2 g (90 % d. Th.)
NMR-Spektrum: $\delta$ = 1,95 6H Ar-$CH_3$ (s)
= 3,0 12H (m)

Im Massenspektrum wurde die theoretische Molmasse 354 bestätigt.

Beispiel 3

Umsetzung von 3,6-Dimethylbenzo-[1.2:4.5]-dicyclobuten mit Zimtsäureethylester (Isomerengemisch)

Die Mischung aus
1 g 3,6-Dimethylbenzo-[1.2:4.5]-dicyclobuten,
20 g Zimtsäureethylester (80 % trans, 20 % cis) und
10 mg 2,6-Di-tert.-butyl-p-kresol
wird 6 Stunden unter Stickstoffabdeckung bei 230 °C gerührt. Nach dem Abkühlen kristallisiert über Nacht der größte Teil des Produkts aus. Der Rest wird nach Abziehen des überschüssigen Zimtsäureethylesters durch Kristallisation des Rückstandes aus Aceton gewonnen.

Ausbeute : 2,7 g (85 % d. Th.)
Smp. : 252 °C

NMR-Spektrum: δ = 7,28   10H (s)
= 3,85   4H (Quartett)
= 3,0   12H
= 2,05   6H (Triplett)
= 0,9   6H (Triplett)

Das scheinbare Triplett bei δ = 2,05 für die Ar-$CH_3$-Gruppen erklärt sich aus der bevorzugten Bildung der vier Isomeren folgender Struktur:

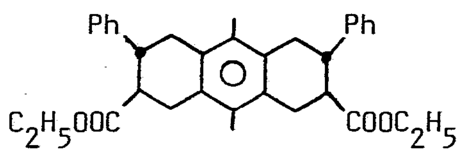
A

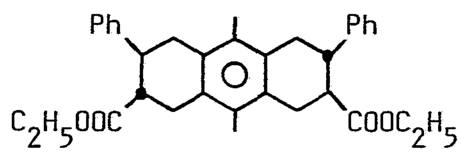
B

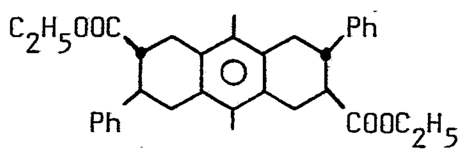
C

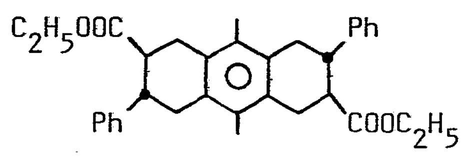
D

Während die chemische Verschiebung für die Ar-$CH_3$-Gruppen der Isomeren A und B verschieden ist, ist dies für die Isomeren C und D nicht der Fall. Aus dem Intensitätsverhältnis der verschiedenen Ar-$CH_3$-Absorptionen kann geschlossen werden, daß das Isomerenverhältnis A + B/C + D = 1 : 1 ist.

Beispiel 4

Wie Beispiel 1, jedoch ohne Zusatz von Hydrochinon.

Ausbeute : 3,0 g (82 % d. Th.)
Smp. : 126 - 140 °C
NMR-Spektrum: δ = 6,8   2H (s)
= 4,1   8H (Quartett)
= 2,9   12H (m)
= 1,25   12H (Triplett)

Patentansprüche:

1. Verfahren zur Herstellung von 1,2,3,4,5,6,7,8-Octahydroanthracenen der allgemeinen Formel

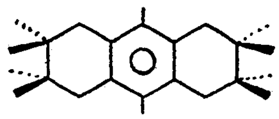,

die wenigstens in zwei der Positionen 2, 3, 6, 7, 9 und 10 substituiert sind,
dadurch gekennzeichnet,
daß man Benzodicyclobutene I der allgemeinen Formel

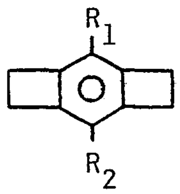,

wobei $R_1$ und $R_2$ jeweils Wasserstoff, ein Halogen oder eine Alkylgruppe mit 1 bis 4 C-Atomen bedeuten,
mit einer oder mehrerer der folgenden Olefinkomponenten bei Temperaturen oberhalb von 100 °C umsetzt:

II trans-Olefine der allgemeinen Formel

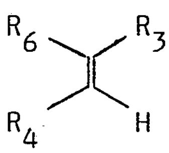,

wobei $R_3$ für einen Rest der Formel $-CO_2-R_5$ oder 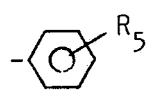 steht und $R_5$ die Bedeutung von Wasserstoff oder eines Alkylrestes mit 1 bis 4 C-Atomen hat, $R_4$ für einen der bereits durch $R_3$ oder $R_5$ definierten Restes steht und $R_6$ die Bedeutung von Wasserstoff oder Methyl hat,

III cis-Olefine der allgemeinen Formel

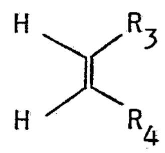 ,

wobei $R_3$ und $R_4$ die bereits vorstehend definierte Bedeutung haben, und

IV cyclische Olefine der allgemeinen Formel

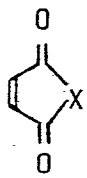 mit X = O, NPh, $NR_5$,

wobei $R_5$ die bereits vorstehend aufgeführte Bedeutung hat.

2. Verfahren nach Anspruch 1, dadurch gekennzeichnet, daß das Molverhältnis Benzodicyclobuten/Olefinkomponente (II bis IV) 1 : n mit $2 < n < 100$ beträgt.

3. Verfahren nach den Ansprüchen 1 und 2, dadurch gekennzeichnet, daß man die Reaktion bei Temperaturen zwischen 170 und 250 °C durchführt.

4. Verfahren nach den Ansprüchen 1 bis 3, dadurch gekennzeichnet, daß man die Umsetzung - bezogen auf die eingesetzte Gewichtsmenge Benzodicyclobuten - in Gegenwart von 0,1 bis 5 % eines die radikalische Polymerisation hindernden Stabilisators durchführt.

5. Octahydroanthracene der allgemeinen Formel

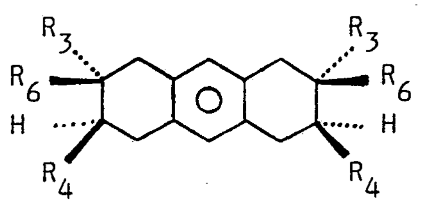

und dessen Stellungsisomere,

erhältlich durch Umsetzung von Benzodicyclobuten mit den trans-Olefinen II gemäß den Ansprüchen 1 bis 4.

6. Octahydroanthracene der allgemeinen Formel

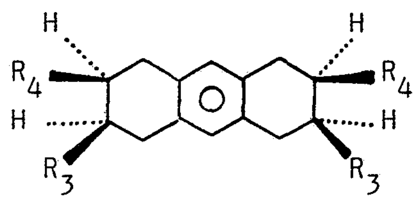

und dessen Stellungsisomere,

erhältlich durch Umsetzung von Benzodicyclobuten mit den cis-Olefinen III gemäß den Ansprüchen 1 bis 4.

7. Octahydroanthracene der allgemeinen Formel

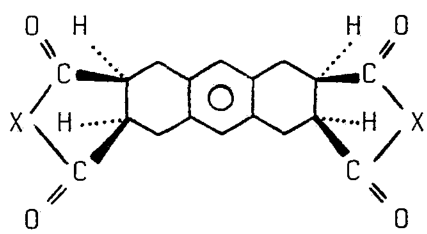

und dessen Stellungsisomere mit X = NPh, $NR_5$,

erhältlich durch Umsetzung von Benzodicyclobuten mit cyclischen Olefinen IV gemäß den Ansprüchen 1 bis 4.